# EUROPEAN PATENT APPLICATION

(11) **EP 1 923 462 A1**
(43) Date of publication of application: **21.05.2008**
(21) Application number: 06807883.1
(22) Date of filing: 25.07.2006
(51) Int. Cl.: C12N 9/10, C12N 9/26, C12P 19/18, C12R 1/645

(54) **NOVEL ENZYME WITH FRUCTOFURANOSIDASE ACTIVITY, WHICH IS USED TO OBTAIN PREBIOTIC OLIGOSACCHARIDES**

(30) Priority: 29.07.2005 ES 200501875
(71) Applicant: Universidad Autónoma de Madrid, 28049 Madrid (ES); Consejo Superior de Investigaciones Cientificas (CSIC), 28006 Madrid (ES)
(72) Inventor: FERNÁNDEZ LOBATO, María, E-28760 Tres Cantos (ES); MACÍAS BORREGO, Isabel, E-28942 Fuenlabrada (ES); MARIN ALBERDI, Dolores, NW6 6SX Londres (GB); LINDE LÓPEZ, Lola, E-28770 Colmenar Viejo (ES); FERNÁNDEZ ARROJO, Lucía, E-28700 San Sebastian de los Reyes - Madrid (ES); PLOU GASCA, Francisco José, E-28049 Madrid (ES)
(74) Representative: Carvajal y Urquijo, Isabel
(86) International application number: PCT/ES2006/000435
(87) International publication number: WO 2007/017537

(57) **Abstract**

The invention relates to a method of obtaining industrially-viable prebiotic oligosaccharides, using a novel extracellular enzyme (fructofuranosidase) of Xanthophyllomyces dendrorhous, which is **characterised in that** it also has transfructosilase activity. The invention also relates to a method of obtaining an enzymatic product with fructofuranosidase activity, as well as the substantially-pure enzyme with said activity. The enzymatic product and the enzyme offer the advantage of having a high performance spectrum, high heat stability and a high specific activity Said prebiotic oligosaccharaides are used in food.

## Description

The present invention is comprised in the field of the biotechnology industry, and particularly the food industry dedicated to obtaining prebiotic oligosaccharides to be used as functional ingredients in dietetic products, dairy products, children's food and food for animals. It is also related to the field of the pharmaceutical and cosmetics industry.

### State of the Art

Carbohydrates, the most abundant biological material occurring in nature, are used as starting elements in a wide variety of industrial processes; therefore, the enzymes involved in their metabolism are of enormous interest from both a basic and a technological point of view.

The field of prebiotic oligosaccharides as functional ingredients in foods has grown considerably in recent years.

The term prebiotic was introduced by Gibson and Roberfroid, who defined prebiotics as non-digestible ingredients in food which beneficially affect the host through a selective stimulation of growth and/or activity of one or of a limited group of bacteria in the colon.

The leading prebiotic molecules on the European Market are fructooligosaccharides (FOS). There are 3 types of fructooligosaccharides described to date. The most well known fructooligosaccharides (¹F series) are those which are currently being marketed and are formed by fructose molecules attached by β,2-1 bonds, with a glucose molecule at the non-reducer end *(*J.W Yun, "Fructooligosaccharides: Occurrence, preparation and application" Enzyme Microb. Technol. 1996, vol. 19, pp. 107-117*),* abbreviated as GFₙ, n typically being comprised between 2 and 4 (kestose, nistose and fructosylnistose). The production of the second type of fructooligosaccharides (⁶F series), in which the fructose molecules are attached by β,2-6 bonds, with a glucose molecule at the non-reducer end is being intensely investigated. These FOS are usually obtained in the form of high molecular weight polymers (levans). Neokestose, a trisaccharide in which a fructose is attached by bonds β,2-6 to the glucose unit in sucrose, is the first representative of the ⁶G series (third type of FOS). The presence of neokestose has been described in *Xanthophyllomyces dendrorhous (Phaffia rhodozyma)* cultures, being the most abundant fructosyltransferase product when this yeast grows in a medium with sucrose *(*S.G. Kilian et al., "Transport-limited sucrose utilisation and neokestose production by Phaffia rhodozyma", Biotechnol. Lett. 1996, vol. 18, pp. 975-980*).*

The aforementioned three types of fructooligosaccharides have prebiotic properties. Due to the type of bond that they have, they resist digestion in the upper gastrointestinal tract, thus preventing being absorbed, becoming an energetic and metabolic substrate for endogenous bacteria of the colon. It has been demonstrated that they have the ability to stimulate the growth of bifidobacteria *(*A. V. Rao, "Dose-response effects of inulin and oligofructose on intestinal bifidogenic effects" J. Nutr. 1998, vol. 80, pp. 1442S-1445S*).* FOS can be obtained by partial hydrolysis of natural polysaccharides (inulin, levan, etc.) or by enzymatic synthesis from sucrose. The profile of products (especially in terms of the average polymerization grade) generated by both methodologies is considerably different, which has an effect on their properties.

The production of ¹F series fructooligosaccharides by means of the hydrolytic route from insulin or by means of the synthetic route by means of the use of sucrose 1-fructosyltransferases (EC 2.4.1.9.), β-fructofuranosidases-invertases- (EC 3.2.1.26) and even by means of levansucrases (EC 2.4.1.10) has been described *(*L.E. Trujillo et al., "Fructo-oligosaccharides production by thr Gluconacetobacter diazotrophicus levansucrase expressed in the methylotrophic yeast Pichia pastoris", Enzyme Microb. Technol. 2001, vol. 28, pp. 139-144*)*. By using some invertases, the synthesis yield that is reached does not exceed 4% of the total of oligosaccharides, whereas with 1-fructosyltransferases the yield that can be reached is much greater (around 50-60%) *(*M. Antosova, M. Polakovic, "Fructosyltransferases: the enzymes catalyzing production of fructooligosaccharides" Chem. Pap. 2001, vol. 55, pp. 350-358*).* Currently, the series ¹F FOS are produced on an industrial level using, in a synthetic function, an invertase of *Aspergillus niger* to generate short-chain oligosaccharides of 3-5 units *(*C. Vannieeuwenburgh et al., "Kinetic studies and mathematical model for sucrose conversion by Aspergillus niger fructosyltransferase under high hydrostatic pressure", Bioprocess Biosyst Eng. 2002, vol. 25, pp. 13-20*).* Other enzymes with fructosyltransferase activity have been described in other fungi such as *Aureobasidium pullulans, Aspergillus oryzae* or *Aspergillus japonicus (*C. S. Chien et al., "Immobilization of Aspergillus japonicus by entrapping cells in gluten for production of fructooligosaccharides". Enzyme Microb. Technol. 2001, vol. 29, pp. 252-257*).*

⁶F series low molecular weight fructooligosaccharides are obtained by means of acid hydrolysis from the levan polymer. In addition, no enzyme has been isolated to date to produce ⁶G series fructooligosaccharides; in the works mentioned to date in the literature whole *Xanthophyllomyces dendrorhous* cells *(Phaffia rhodozyma)* or of different fungi (e.g. *Penicillium citrinum,* immobilized cells) are used *(*Lee et al., "Reaction route for enzymatic production of neofructo-oligosaccharides from sucrose using Penicillium citrinum cells", J. Microbiol. 2001, vol. 39, pp. 331-333*;* Park et al., "Continuous production of neo-fructooligosaccharides by immobilization of whole cells of Penicillium citrinum", Biotechnol. Lett. 2005, vol. 27, pp. 127-130*).*

The effects of FOS on the person who consumes them can be quite varied: reducing of episodes of diarrhea caused by rotavirus; improvement of lactose intolerance symptoms; control of constipation by a fecal mass increase; increased calcium absorption, and consequently a reduced risk of osteoporosis; decreased mutagenic capability of certain microbial enzymes such as nitro-reductase, associated with colon cancer; possible reduction of diseases related to dyslipidemia, etc.

The yeast *Xanthophyllomyces dendrorhous* (also called *Phaffia rhodozyma)* is currently used in the biotechnological industry to produce astaxanthin, a carotenoid which has shown great effectiveness in the pigmentation of salmonids, crustaceans and in poultry farming. X. *dendrorhous* accumulates this carotene naturally and furthermore freely, which facilitates preparing the pigment. This makes it the microorganism in which the production is globally more profitable than in any other. It is currently marketed as "Natupink" by Gist-Brocades (EP 551676 B1). Hoffman-La Roche (Basel, Switzerland) has marketed the synthetic astaxanthin called "Carophyll pink". Antibióticos S.A. has developed the process for producing astaxanthin by means of fermenting selected strains of *X. dendrorhous* (ES 2203315 A1).

The commercial interest in astaxanthin has generated considerable advancement in the genetic and molecular knowledge of the producer organism. The genes involved in producing astaxanthin have been characterized, but some of the genes carbon sources, such as those responsible for the synthesis of an endo-beta-1,3(4)-glucanase, a protease *(*M.L. Bang et al., "Cloning and characterization of an endo-beta-1,3(4)-glucanase and aspartic protease from Phaffia rhodozyma CBS 6938", Appl. Microbiol. Biotechnol. 1999, vol. 51, pp. 215-22*)*, or glyceraldehyde-3-phosphate dehydrogenase *(*J.C. Verdoes et al., "Molecular characterization of the glyceraldehyde-3-phosphate dehydrogenase gene of Phaffia rhodozyma", Yeast 1997, vol. 13, pp. 1231-42*)* have also been characterized. A 240 kDa extracellular protein with beta-amylase activity has been partially purified and characterized *(*A. Díaz et al., "Production and partial characterization of a beta-amylase by Xanthophyllomyces dendrorhous", Lett. Appl. Microbiol. 2003, vol. 36, pp. 203-7*).* A patent for an extracellular α-glucosidase with high-spectrum glycosyltransferase activity capable of generating malt and isooligosaccharides using maltase has been filed (P200402994, UAM-CSIC). Some proteins, such as invertase associated to the cell fraction or urease, have also been studied in this organism *(*D.S. Persike et al., "Invertase and urease activities in the carotenogenic yeast Xanthophyllomyces dendrorhous (formerly Phaffia rhodozyma)", Bioresour Technol. 2002, vol. 82(1), pp. 79-85*).*

Whole X. *dendrorhous* cells have been indicated for the production of neokestose (CAS Registry Number 3688-75-3), a prebiotic trisaccharide obtained from sucrose. A suspension of cells of this organism (phosphate-citrate buffer) was used for the reaction. Maximum production values for the oligosaccharide were obtained at 25°C and 11% sucrose. A maximum of 75.1 g/l of neokestose was obtained *(*S.M. Kritzinger et al., "The effect of production parameters on the synthesis of the prebiotic trisaccharide, neokestose, by Xanthophyllomyces dendrorhous (Phaffia rhodozyma)", Enzyme and Microbial Technology 2003, vol. 32, pp. 728-37*).*

Given the industrial importance of prebiotic oligosaccharides, it is desirable to provide enzymes and industrially viable processes for obtaining them.

### Description of the Invention

The present invention relates to a novel extracellular *Xanthophyllomyces dendrorhous* enzyme characterized by showing fructosyltransferase activity and useful for obtaining oligosaccharides. Thus, one aspect of the invention relates to a process of obtaining an enzymatic product with fructosyltransferase activity which comprises culturing *X. dendrorhous* cells in an appropriate medium and under suitable conditions. A person skilled in the art will choose the culture mediums and conditions, such as pH, temperature and stirring for culturing X. *dendrorhous,* by means of conventional methods. Culture examples will be described in detail below.

Fructosyltransferase activity is associated to β-D-fructofuranosidase or invertase (EC 3.2.1.26) (IUBMB Enzyme Nomenclature, CAS Registry Number 9001-42-7), enzymes which hydrolyze sucrose into fructose and glucose.

The crude enzymatic product resulting from the previous process of the invention can now be used on the industrial level to obtain oligosaccharides without requiring subsequent separation or purification steps. In a particular embodiment of the invention, the process further comprises the step of recovering the enzymatic product from the culture medium and/or from the cells, as the enzyme object of the invention is released extracellularly. Thus, both the suspension of *X. dendrorhous* cells with the suitable culture medium so that the fructofuranosidase activity is expressed as well as the cell-free fraction are understood as the enzymatic product in this description. A person skilled in the art will choose the starting enzymatic product most suited to each industrial process, i.e. a crude product or product having a higher or lower level of purification, by means of conventional methods.

In another particular embodiment of the invention the X. *dendrorhous* cells belong to a strain selected from the group consisting of ATCC:MYA-131, ATCC 24230, ATCC 24202. The ATCC: MYA-131 strain was deposited in the American Type Culture Collection (12301 Parklawn Drive, Rockville, Md. 20852, USA).

Another aspect of the invention relates to the enzymatic product with fructosyltransferase activity obtainable by means of the process hereinbefore defined. The enzymatic product of the invention is very efficient in degrading sucrose and also acts on oligosaccharides such as 1-kestose, nistose, raffinose and palatinose. In a particular embodiment of the invention, the enzymatic product of the invention is characterized in that the fructofuranosidase activity has low substrate specificity, acting on sucrose, 1-kestose, nistose, raffinose and palatinose. In another particular embodiment, the fructofuranosidase activity of the enzymatic product has a maximum value in the pH interval between 5.5 and 7.5 at 42 °C, and in a temperature interval between 50 and 75°C.

In addition to fructofuranosidase activity, the enzymatic product of the invention has fructosyltransferase activity in the presence of high concentrations of sucrose. In a particular embodiment, the products resulting from the fructosyltransferase activity are of the ⁶G (particularly trisaccharide neokestose) and ¹F (particularly 1-kestose) series fructooligosaccharides.

Another aspect of the invention relates to a process for obtaining a substantially pure enzyme with fructofuranosidase/fructosyltransferase activity, comprising the steps of: (a) obtaining an enzymatic product with fructofuranosidase/fructosyltransferase activity by means of culturing X. *dendrorhous* cells in an appropriate medium and under suitable conditions; (b) recovering the enzymatic product from the culture medium and/or from the cells; and (c) purifying the enzymatic product until obtaining a substantially pure enzyme with fructofuranosidase/fructosyltransferase activity. In a particular embodiment of the invention, the X. *dendrorhous* cells belong to a strain selected from the group consisting of ATCC:MYA-131, ATCC 24230, ATCC 24202.

The invention also relates to a substantially pure enzyme with fructofuranosidase/fructosyltransferase activity which can be obtained by the defined process. Conventional purification methods could be used to obtain the enzyme of the invention. An example of a purification method is described in detail in this description.

The indicated substrate specificity features for the enzymatic product of the invention are also attributed to the purified enzyme. Fructosyltransferase activity is also characteristic of the purified enzyme. Furthermore, in a particular embodiment, the enzyme is characterized by having a molecular weight of approximately 214 kDa determined by molecular filtration. The purified enzyme in the present invention has been characterized kinetically and by mass spectrometry. This data is included below in this description.

Several positive aspects of the enzymatic product and of the enzyme of the invention are that they have a high action spectrum and a high specific activity, making them suitable candidates for hydrolyzing or modifying oligosaccharides. Another important aspect on the industrial level is that the enzymatic product and the enzyme are stable for long reaction times (e.g. 160 h) at a temperature of approximately 45°C, as is illustrated in the detailed description below.

The present invention involves an industrially viable process of obtaining oligosaccharides. Thus, another aspect of the invention relates to a process for obtaining oligosaccharides which comprises allowing the previously defined enzymatic product or purified enzyme to act on one or several glucidic substrates. A person skilled in the art will choose the culture mediums, the substrates and the reaction conditions to carry out the process by means of conventional methods. Furthermore, the enzyme or *X. dendrorhous* cells producing the enzyme can be used as such or immobilized, physically or chemically coupled to a carrier material. Reuse of the enzyme or the cells is thus allowed. Preparation examples are included below in this description.

### Enzymatic Characterization

### A) Expression of X. dendrorhous fructofuranosidase activity.

The production of fructofuranosidase activity was analyzed in X. *dendrorhous* cultures grown in yeast minimal medium: YNB (0.7% Yeast Nitrogen Base w/o Amino acids, DIFCO) (w/v), 2% carbon source. The cultures were carried out in glass flasks incubated at a temperature comprised between 21-25°C and constant orbital shaking of 100-250 rpm. The optimal growth conditions were 23°C and 160 rpm.

The cell-free fraction was obtained by centrifugation (F-0) of an X. *dendrorhous* culture grown at 23°C and 160 rpm. Fructofuranosidase activity was assayed in this fraction by assessing the release of glucose on different substrates. A colorimetric assay and standard methodology were used. The released glucose was quantified using the glucose oxidase-peroxidase coupled reaction: 0.4 ml of the solution to be assayed was mixed with 0.1 ml of A:B solution (20:1) (A: 0.85 U/ml glucose oxidase, 0.40 U/ml peroxidase in sodium phosphate buffer pH 5; B: 0.6% o-dianisidine). It was incubated for 30 minutes at 37 °C and spectrophotometrically quantified at 450 nm. A standard glucose curve (1 to 100 µg/ml) was used. The fructofuranosidase activity unit is defined in µg/ml min of glucose assessed in the described conditions. Figure 1 shows the results obtained when the MYA-131 strain is used and the assay is performed on sucrose.

### B) Characterization of X. dendrorhous fructofuranosidase activity after culturing and centrifuging the cell-free fraction.

The cell-free fraction, obtained by centrifuging an *X. dendrorhous* culture in maltose minimal medium, grown to an optical density of 2.37 ODU₆₆₀ₙₘ, was assayed on different substrates. The assay results are shown in Table 1. No activity was detected when leucrose, maltose, maltotriose or maltoheptose was used as a substrate.

| TABLE 1. Fructofuranosidase activity of the extracellular fraction of *X. dendrorhous* on different carbohydrates | |
|---|---|
| Substrate | Specific activity (mU/µg) |
| sucrose | 62140 |
| raffinose | 335 |
| 1-kestose | 1675 |
| nistose | 502 |
| palatinose | 217 |

### C) Purification of the enzyme with X. dendrorhous fructofuranosidase activity.

1 liter of extracellular fraction of X. *dendrorhous* with fructofuranosidase activity of 439.2 U/ml was used to purify the enzyme. The following method was used:
   1) Concentrating the extracellular fraction using a double tangential flow filtration system (30 kDa and 50 kDa filters) followed by dialysis against 20 mM HCI-Tris pH 7 (buffer A) for 2 hours at a temperature of 4°C. 20 ml of concentrate were obtained with an activity of 6410 U/ml (F-1).
   2) Ion exchange chromatography at pH 7. The sample was applied to a 10 ml DEAE-Sephacel ion exchange column equilibrated with buffer a. Elution was performed using a 0 to 0.150 M NaCl gradient. The fraction eluted at 0.1 M of salt was dialyzed against 20 mM sodium acetate pH 5 (buffer B) for 2 hours at 4°C (F-2).
   3) Ion exchange chromatography at pH 5. The sample was applied to the DEAE-Sephacel ion exchange column equilibrated with buffer B and eluted using a discontinuous gradient of NaCl of 0.05, 0.1 and 0.2 M of salt. The fructofuranosidase activity assessed as previously indicated eluted at 0.1 M of salt and was 4326 U/ml (F-3).

Purification was assessed by analyzing the proteins present after each of the purification steps (F-1, F-2 and F-3) in SDS-PAGE polyacrylamide gels and staining. The obtained results are shown in the Figure 2.

### D) Determination of the molecular weight of the X. dendrorhous enzyme with fructofuranosidase activity by molecular filtration.

Molecular exclusion chromatography was used to determine the molecular weight of fructofuranosidase activity. A 70 ml Sephadex G-100 (SIGMA) column equilibrated with 500 ml of 50 mM sodium phosphate pH 5.5 was used at a rate of 0.6 ml/min. 1.5 ml of an enzymatic preparation with a specific activity of 297 U/ml on sucrose was applied. 3 ml fractions were collected. Blue Dextran (2000 kDa), catalase (240 kDa), albumin (67 kDa) and ovalbumin (45 kDa) were used as molecular weight markers, and they eluted at 54, 63, 84 and 90 ml, respectively. The entire process was performed at 4°C. The fructofuranosidase activity was assayed on sucrose using the previously described methodology. Activity was detected in the fraction eluted at 66 ml, which corresponds with a protein with a molecular weight of 214.5 ± 2% kDa.

The fructofuranosidase purified to homogeneity according to the described process has a molecular weight calculated by molecular filtration (G-100) of 214.5 ± 2% kDa.

### E) Characterization of the X. dendrorhous enzyme with fructofuranosidase activity by mass spectrometry

The protein was digested with trypsin (Promega Trypsin-TPCK) under standard digestion conditions (A. Shevchenko et al., "Mass spectrometric sequencing of proteins silver-stained polyacrylamide gels", Anal Chem. 1996 vol. 68(5), pp. 850-8*).* The supernatant of digestion with trypsin was analyzed in a Bruker Autoflex MALDI-TOF (matrix-assisted laser desorption ionization/time-of-flight) type mass spectrometer equipped with a non-linear reflector, with a UV-nitrogen laser at 337 nm, with 3 nanosecond pulses following the standard methodology using HCCA (α-cyano-4-hydroxycinnamic acid) as a matrix under saturation and 0.1% trifluoroacetic acid and 33% acetonitrile (v/v) conditions. The obtained result is shown in Figure. 3. This spectrum was used as a "peptide fingerprint" for identifying proteins in databases using on-line search engines (Mascot, Profound), based on the relative intensity (r.i.) of ionized tryptic peptides against the mass/charge (m/z) thereof. The isolated protein is a novel molecule that has never before been described since MALDI-TOF profiles coinciding with proteins known by their amino acid sequence, or the corresponding protein deduced by the DNA sequence, have not been found.

### F) Characterization of the fructofuranosidase activity of the purified enzyme.

The hydrolytic activity of the enzyme purified to homogeneity (F-3) following the described process was assayed on different substrates. The maximum level of activity is obtained on sucrose. No activity was observed on leucrose, maltose, maltotriose and maltoheptose. The obtained results are shown in Table 2.

| TABLE 2. Fructofuranosidase activity of the *X. dendrorhous* F-3 fraction on different substrates. The assays were performed using purified protein and a 1% concentration for all the assayed substrates. | |
|---|---|
| Substrate | Specific activity (U/µg) |
| sucrose | 675 |
| raffinose | 8 |
| 1-kestose | 41 |
| nistose | 13 |
| palatinose | 5 |

The fructofuranosidase activity was assayed at different pH and temperatures. Maximum activity levels were obtained in a pH range comprised between 5.5 and 7.5 units and a temperature of 50-75 °C. Figure 4 shows the obtained results.

The kinetic characterization of the enzyme purified according to the described process was performed on sucrose. The data is included in Table 3. The assays were performed in 20 minutes and 20 µl of purified enzyme for each reaction according to the described process.

| TABLE 3. Kinetic constants of *X. dendrorhous* fructofuranosidase activity. Standard error of the mean ±10 | | | | |
|---|---|---|---|---|
| Substrate | Vₘₐₓ (µg glucose min⁻¹) | k_{cat} (min⁻¹) | Kₘ (mM) | k_{cat}/Kₘ (mM⁻¹ min⁻¹) |
| sucrose | 947 | 141 | 4 | 35.2 |

### G) Fructosyltransferase activity of the X, dendrorhous enzyme.

A study was carried out using a high concentration of sucrose (600 g/I), conditions which can favor the formation of glycosidic bonds in detriment of hydrolysis. The profile of the formed products is shown in Figure. 5. It can be seen that the X. *dendrorhous* enzyme shows both hydrolysis activity and transfer activity. On one hand, fructose (peak 1) and glucose (peak 2) are formed as hydrolytic products. On the other hand, two trisaccharides are formed: a majority product (peak 4), which is most likely neokestose [β-D-Fru-(2→6)-α-D-Glu-(1→2)--D-Fru] (since it is the majority transfructosylation product described in X. *dendrorhous* cultures) and another minor product (peak 5), identified as 1-kestose [α-D-Glu-(1→2)-β-D-Fru(1→2)-β-D-Fru]. Small amounts of different compounds (peaks 6-8) which, taking into account the retention time in the column, are probably tetrasaccharides not yet identified, are furthermore observed. The sucrose which has not yet reacted corresponds to peak 3. The scheme of the first reaction step is shown in Figure. 6

Table 4 shows the composition (in g/l) of the oligosaccharides present in the reaction mixture over 160 hours of incubation a 45°C. The novel enzyme characterized in this work continues to maintain fructosyltransferase activity after 160 hours at 45°C. The total percentage (w/w) of fructooligosaccharides after 160 hours of reaction was 14.5%, a value which refers to the total weight of carbohydrates in the medium.

| TABLE 4. Composition of the reaction mixture over time after incubating sucrose with the purified *X. dendrorhous* enzyme (F-3) at 45°C. Reaction conditions: 600 g sucrose/I in 0.2 M sodium acetate (pH 5.6), 150 rpm. The assay was performed with 5 U/ml of biocatalyst. HPLC analysis using a Waters delta 500 pump, Lichrospher 100-NH2 column (Merck), 250 x 4.6 mm, 75:25 v/v acetonitrile:water, 0.7 ml/min, 25°C, Varian refraction index detector. Name of the compounds: 1, fructose; 2, glucose; 3, sucrose [a-D-Glu-(1→2)-β-D-Fru]; 4, neokestose [β-D-Fru-(2→6)-α-D-Glu-(1→2)-β-D-Fru]; 5, 1-kestose [a-D-Glu-(1→2)-β-D-Fru(1→2)-β-D-Fru]. | | | | | | |
|---|---|---|---|---|---|---|
| Reaction time (h) | Composition of the reaction mixture (grams/liter) | | | | | Percentage (w/w) of FOS^{a} |
| | 1 | 2 | 3 | 4 | 5 | |
| 0 | 0 | 0 | 600 | 0 | 0 | 0 |
| 24 | 19.0 | 37.8 | 513.4 | 20.5 | 9.2 | 5.0 |
| 140 | 63.5 | 106.8 | 357.5 | 47.4 | 24.8 | 12.0 |
| 160 | 65.1 | 109.3 | 338.9 | 53.3 | 33.5 | 14.5 |

| | | | | | | |
|---|---|---|---|---|---|---|
| ^{a} Percentage relating to the total weight of sugars | | | | | | |

Table 5 shows the composition (in g/l) of the oligosaccharides present in the reaction mixture over 160 hours of incubation at 60°C. The total percentage (w/w) of fructooligosaccharides after 160 hours of reaction was the 9.7%, value relating to the total carbohydrates in the medium. The novel enzyme characterized in this work is partially inactivated after 160 hours at 60°C. Thus, the addition of a new biocatalyst load (5 U) to the reaction mixture after 140 hours of reaction allows increasing the percentage of FOS in the reaction mixture up to a value of 11.6%

| TABLE 5. Composition of the reaction mixture over time after incubating sucrose with the purified *X. dendrorhous* enzyme (F-3) at 60°C. Reaction conditions: 600 g sucrose/I in 0.2 M sodium acetate (pH 5.6), 150 rpm. The assay was performed with 5 U/ml of biocatalyst. HPLC analysis using a Waters delta 500 pump, Lichrospher 100-NH2 column (Merck), 250 x 4.6 mm, 75:25 v/v acetonitrile:water, 0.7 ml/min, 25°C, Varian refraction index detector. Name of the compounds: 1, fructose; 2, glucose; 3, sucrose [α-D-Glu-(1→2)- p-D-Fru]; 4, neokestose [β-D-Fru-(2→6)-α-D-Glu-(1→2)- p-D-Fru]; 5, 1-kestose [α-D-Glu-(1→2)- βD-Fru(1→2)- p-D-Fru]. | | | | | | |
|---|---|---|---|---|---|---|
| Reaction time (h) | Composition of the reaction mixture (grams/liter) | | | | | Percentage (w/w) of FOS^{a} |
| | 1 | 2 | 3 | 4 | 5 | |
| 0 | 0 | 0 | 600 | 0 | 0 | 0 |
| 24 | 36.8 | 53.2 | 474.0 | 25.8 | 10.3 | 6.0 |
| 140 | 40.9 | 79.3 | 424.2 | 34.3 | 21.3 | 9.3 |
| 160 | 41.2 | 84.3 | 416.1 | 36.5 | 21.9 | 9.7 |

| Addition of a new catalyst load (5 U) after 140 hours | | | | | | |
|---|---|---|---|---|---|---|
| 160 | 56.5 | 119.5 | 358.4 | 41.6 | 23.9 | 10.9 |
| 186 | 76.0 | 131.2 | 323.1 | 43.9 | 25.8 | 11.6 |

| | | | | | | |
|---|---|---|---|---|---|---|
| ^{a} Percentage relating to the total weight of sugars | | | | | | |

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following detailed description, examples and drawings are provided by way of illustration and are not intended to be limiting of the present invention.

### Description of the Drawings

Figure 1 shows the production of extracellular fructofuranosidase activity through out the MYA-131 X. *dendrorhous* culturing. The yeast was grown in 2% maltose minimal medium at 23 °C and constant orbital shaking at 160 rpm for 145 hours. Growth of the culture in ODU₆₆₀ₙₘ (squares) and fructofuranosidase activity assessed in the extracellular medium in U/ml (triangles), reached at the times indicated, are represented. The activity was assayed on 1 % sucrose.
Figure 2 shows the result of the SDS-PAGE (8%) analysis of the proteins present throughout the purification process. 500 µl of the indicated fractions were concentrated up to a final volume of 10 µl using an Amicon Microcon YM-10 system of and were analyzed in 8% polyacrylamide gels. The gel was stained with silver salts following the standard methodology. M: Molecular weight markers; 1, 2 and 3: F-1, F-2 and F-3, respectively.
Figure 3 is the result of the MALDI-TOF mass spectrometry analysis of the protein purified to homogeneity. The relative intensity (r.i.) of the ionized tryptic peptides is shown against the mass/charge (m/z) thereof. The supernatant of digestion with trypsin was analyzed in a Bruker Autoflex MALDI-TOF mass spectrometer equipped with a reflector, using (α-cyano-4-hydroxycinnamic acid) as a matrix under saturation conditions.
Figure 4 indicates the enzymatic activity (A) according to the pH and temperature, showing the corresponding maximum values. In Figure 4A, fructofuranosidase activity was assessed at different pH values (3-8 pH units) and temperatures (25-90 °C). The assay was performed on sucrose using a pure protein solution. 100% corresponds with an activity of 3045 µg glucose/ml min. An assay temperature of 42 °C and sodium citrate buffer, sodium phosphate and Tris (all at 50 mM) were used in the pH assessment for the pH intervals of 3-5, 5.5-7 and 8, respectively. In Figure 4B, the temperature assay was conducted in 50 mM sodium phosphate, pH 7.
Figure 5 shows the profile of products obtained after incubating sucrose at a concentration of 600 g/I with the purified X. *dendrorhous* enzyme (F-3). The reaction conditions, HPLC analysis conditions and names of the compounds are the same as in Table 5. The chromatographic analysis corresponds to 120 h of reaction.

### Embodiments

### Example 1: Production of fructofuranosidase throughout X. dendrorhous cultures, strains ATCC 24202 and ATCC 24230, grown in minimal maltose medium.

*X. dendrorhous* was cultured from ATCC 24202 and ATCC 24230 strains in 100 ml of yeast minimal medium supplemented with maltose (MMM): YNB (0.7% Yeast Nitrogen Base w/o Amino acids (w/v), DIFCO), 2% maltose (w/v) to produce fructofuranosidase. The cultures continued for 80 hours. Cell growth was spectrophotometrically assessed following the absorbance of the culture at an optical density of 660 nm (ODU₆₆₀). 250 ml glass flasks, a temperature of 23°C and constant orbital shaking were used. The stationary phase was reached 40 hours after growth for ATCC 24202 and 50 hours for ATCC 24230, at 3.9 and 2.9 ODU₆₆₀, respectively. 1 ml of the culture was taken every 3-4 hours and the cells were removed by centrifugation, 2-3 minutes in a microcentrifuge (16000xg).

### Example 2: Use of the enzyme for producing glucose from sucrose with the minimal medium supernatant

The culture supernatants of Example 1 were used to release glucose from sucrose due to the action of the fructofuranosidase activity of the supernatant in which the extracellular enzyme is located. To that end, 20 µl ml of the cell-free fraction, 0.48 ml of 50 mM sodium phosphate buffer pH 5.5 and 0.5 ml of 1% sucrose in this same buffer were mixed and incubated at 42°C for 60 minutes. Considerable fructofuranosidase activity levels were obtained from the beginning of the logarithmic growth phase of the cultures until the end of the stationary phase. Maximum activity levels (804 U/ml for ATCC 24202 and 727 U/ml for ATCC 24230) were obtained in the stationary phase and remained constant for at least 30-40 culture hours.

### Example 3: Production of fructofuranosidase throughout X. dendrorhous cultures grown in a medium with succinate.

*X. dendrorhous* MYA-131 grown in 100 ml of succinate-supplemented medium (YMS): 0.3% yeast extract (w/v), 0.3% malt extract (w/v), 0.5% peptone (w/v), 1 % succinic acid (w/v) pH 4.5, was cultured to produce fructofuranosidase. The culture continued for 150 hours. Cell growth was carried out and assessed as in the previous example. The stationary phase was reached after 55 hours of growth, at 4.5 ODU₆₆₀. The cell-free fraction was obtained as in Example 1 every 3-7 hours.

### Example 4: Use of the enzyme for producing glucose from sucrose with succinate medium supernatant

The culture supernatant of Example 3 was used to release glucose from sucrose due to the action of the fructofuranosidase activity of the supernatant in which the extracellular enzyme is located, as in Example 2. Assessable levels of fructofuranosidase activity were obtained from half of the logarithmic growth phase until the end of the curve, for about 120 hours of culture. The maximum activity levels (103 U/ml) were obtained at 4.2-4.5 ODU₆₆₀.

### Example 5: Production of fructofuranosidase throughout X. dendrorhous cultures grown in a rich medium

The production of fructofuranosidase through an X. *dendrorhous* MYA-131 culture grown in 100 ml of yeast rich medium (YEP) supplemented with maltose (YEPM): 1% yeast extract (DIFCO) (w/v), 2% bactopeptone (DIFCO) (w/v), 2% maltose (w/v), continued for 90 hours. A 250 ml glass flask, a temperature of 24°C and constant orbital shaking of 200 rpm were used. Cell growth was assessed as in Example 1. The stationary phase was reached after 50 hours of growth, at 5.8 ODU₆₆₀. The cell-free fraction was obtained as in Example 1 every 3-4 hours.

### Example 6: Use of the enzyme to produce glucose from sucrose with rich medium supernatant

The culture supernatant of Example 5 was used to release glucose from sucrose due to the action of the fructofuranosidase activity of the supernatant in which the extracellular enzyme is located as in Example 2. Assessable levels of fructofuranosidase activity were obtained from half of the logarithmic growth phase of the culture up to the end of the curve, for about 90 hours of culture. The maximum activity values (320 U/ml) were obtained at 4.2-4.5 ODU₆₆₀.

### Example 7: Stability of X. dendrorhous fructofuranosidase at 60°C

The extracellular fraction of an X. *dendrorhous* culture was concentrated 50 times using a tangential flow filtration system (50 kDa filters) followed by dialysis against 20 mM sodium phosphate pH 7 for 2 hours at a temperature of 4°C. An enzymatic preparation with an activity of 6410 U/ml was obtained. The preparation was maintained at 60°C for 24 hours. 20 µl of the preparation were taken every 1-4 hours and frozen at a temperature of -20°C. The fructofuranosidase activity on sucrose was assessed in all the samples obtained as in Example 2 (42°C, 60 min). After 1 hour at 60°C 5120 U/ml of fructofuranosidase activity were obtained, approximately 80% of the starting units. This value remained constant over the 24 hours of assay.

### Example 8: Formation of fructooligosaccharides at 45°C from sucrose catalyzed by of X. dendrorhous fructofuranosidase.

A solution with a high concentration of sucrose (600 g/I) in 0.2 M sodium acetate buffer pH 5.6 was prepared. 5 U/ml of fructofuranosidase (a unit U is the enzymatic activity corresponding to the release of a micromole of reducer sugars per minute using sucrose 100 g/I as a substrate in 0.2 M buffer sodium acetate pH 5.6 at 60°C) were added. The reaction mixture was incubated for 160 hours at 40°C, with orbital shaking at 200 rpm. Aliquots were extracted at different times, incubated for 5 minutes at 80°C to inactivate the enzyme, diluted 1:4 (v/v) with water, centrifuged for 5 min at 6000 rpm in an Eppendorf tube with a 0.45 µm filter and analyzed by HPLC liquid chromatography. The profile of the products formed can be seen in Figure. 5. It can be seen that X. *dendrorhous* fructofuranosidase shows transfer activity (transfructosylation). Two trisaccharides are obtained: a majority product which is most likely neokestose [β-D-Fru-(2→6)-α-D-Glu-(1→2)--D-Fru] and another minor product identified as 1-kestose [α-D-Glu-(1→2)-β-D-Fru(1→2)-β-D-Fru]. At the end time (160 hours), the composition of the system was: 10.5% fructose, 17.6% glucose, 54.6% sucrose, 17.3% fructooligosaccharides (tri- and tetrasaccharides).

## Claims

1. A process for obtaining an enzymatic product with fructofuranosidase activity, which comprises culturing *Xanthophyllomyces dendrorhous (Phaffia rhodozyma)* cells in a maltose-based yeast medium at a temperature of 23°C and constant orbital shaking of 160 rpm.

2. A process according to claim 1, which comprises culturing *Xanthophyllomyces dendrorhous* cells in a yeast minimal or rich medium based on different carbon sources (maltose, derivatives of the disaccharide, succinate) at a temperature comprised between 21 and 25°C and in a range of constant orbital shaking comprised between 100 and 250 rpm.

3. A process according to claims 1 and 2, further comprising the step of recovering the enzymatic product from the culture medium and/or from the cell suspension.

4. A process according to any of claims 1-3, wherein the *Xanthophyllomyces dendrorhous* cells belong to a strain selected from the group consisting of ATCC: MYA-131, ATCC 24230, ATCC 24202.

5. An enzymatic product with fructofuranosidase activity which can be obtained by the defined process in any of claims 1-4.

6. An enzymatic product according to claim 5, **characterized in that** the fructofuranosidase activity has low substrate specificity, acting on sucrose, raffinose, 1-kestose, nistose, and palatinose.

7. An enzymatic product according to any of claims 5 and 6, **characterized in that** it has no fructofuranosidase activity on leucrose, maltose, maltotriose, and maltoheptose.

8. An enzymatic product according to any of claims 5-7, wherein the fructofuranosidase activity shows maximum activity in the pH interval between 5.5 and 7.5 at 42°C, and in a temperature interval of 50 to 75°C.

9. An enzymatic product according to any of claims 5-8, **characterized in that** it has fructosyltransferase activity in presence of one or several glucidic substrates.

10. An enzymatic product according to claim 9, **characterized in that** the glucidic products are fructooligosaccharides.

11. An enzymatic product according to claim 10, wherein the products resulting from fructosyltransferase activity are oligosaccharides with β-1,2, and β-2,6 bonds.

12. An enzymatic product according to claim 11, wherein the products resulting from fructosyltransferase activity are basically neokestose, 1-kestose

13. A process for obtaining oligosaccharides which comprises allowing the enzymatic product defined in any of claims 5-12 to act on one or several glucidic substrates.

14. A process of obtaining a substantially pure enzyme with fructofuranosidase activity from the enzymatic product obtained by the process according to claims 1 and 2, comprising the additional step of purifying the enzymatic product using, among other techniques, tangential flow filtration, molecular filtration and/or ion exchange chromatography, until obtaining the substantially pure enzyme.

15. A process according to claim 14, wherein the *Xanthophyllomyces dendrorhous* cells belong to a strain selected from the group consisting of ATCC: MYA-131, ATCC 24230, ATCC 24202.

16. A substantially pure enzyme with fructofuranosidase activity which can be obtained by the process defined in any of claims 14 and 15.

17. An enzyme according to claim 16, **characterized in that** the fructofuranosidase activity has low substrate specificity, acting on sucrose, raffinose, 1-kestose, nistose and palatinose.

18. An enzyme according to any of claims 16 and 17, **characterized in that** it does not have fructofuranosidase activity on leucrose, maltose, maltotriose, maltoheptose.

19. An enzyme according to any of claims 16-18, wherein the fructofuranosidase activity shows maximum activity in the pH interval between 5.5 and 7.5 at 42°C, and in a temperature interval between 50 and 75°C.

20. An enzyme according to any of claims 16-19, **characterized in that** it has fructosyltransferase activity in the presence of one or several glucidic substrates.

21. An enzyme according to claim 20, **characterized in that** the glucidic products are fructooligosaccharides.

22. An enzyme according to claim 21, wherein the products resulting from fructosyltransferase activity are oligosaccharides with β-1,2, and β-2,6 bonds.

23. An enzyme according to claim 22, wherein the products resulting from fructosyltransferase activity are basically neokestose and 1-kestose.

24. An enzyme according to any of claims 16-23, **characterized by** having a molecular weight calculated by molecular filtration of approximately 214 kDa in natural conditions, and 170 kDa in denaturing conditions.

25. A process of obtaining oligosaccharides which comprises allowing the enzyme defined in any of claims 16-24 to act on one or several glucidic substrates.
